# EUROPEAN PATENT APPLICATION

(11) **EP 3 067 090 A1**
(43) Date of publication of application: **14.09.2016**
(21) Application number: 15158397.8
(22) Date of filing: 10.03.2015
(51) Int. Cl.: A61N 1/05, H01B 1/00, A61N 1/04, A61N 1/08

(54) **Electrode device for electrodiagnosis and/or electrotherapy and implant comprising an electrode device**

(71) Applicant: BIOTRONIK SE & Co. KG, 12359 Berlin (DE)
(72) Inventor: Rump, Jens, 12049 Berlin (DE)
(74) Representative: Galander, Marcus

(57) **Abstract**

The invention relates to an electrode device (10) for cardiological or neurological electrodiagnosis and/or electrotherapy, comprising at least one elongated electrode body (20), which has a proximal side (18) and a distal side (24), wherein the electrode body (20) comprises at least one electrode (30, 40) on the distal side (24) for establishing electrical contact to the tissue and/or blood during use, wherein at least one of the electrodes (30, 40) is coated, at least in sections, with a dielectrically anisotropic material (46).

## Description

The invention relates to an electrode device for electrodiagnosis and/or electrotherapy and to an implant comprising an electrode device according to the preambles of the independent claims.

The capability to perform a reliable analysis of the excitation pathways in the heart is very important in order to detect pathological changes in the human heart. To this end, electrodes are placed in the ventricle and/or atrium in order to locally trigger the course of the excitation over time. Due to the different amplitudes of the excitation signals in the ventricle and the atrium, erroneous assignments can occur when the electrical fields induced by the excitation reach a significant amplitude over a large region of the heart. In particular, ventricular signals can also be detected by an electrode placed in the atrium. These so-called far-field signals must also be detected as such by the active implant and must be filtered out of the analysis of the useful signals.

Although far-field signals can be suppressed by shortening the pole separation, this also reduces the useful signals. As a result, it is possible that signals to be detected are located below the sensitivity threshold of the implant and are detected not at all or erroneously. Information on the morphology of the excitation is therefore lost and the reliability is adversely affected. In addition, reducing the pole separation changes the mechanical properties of the electrodes and a flexible range between the tip electrode and the ring electrode is no longer ensured.

Electrode devices for implants for electrodiagnosis and/or electrotherapy are known, for example from WO 2005/053555 A1. These are used in the field of electrophysiology, in particular for the detection and treatment of conduction defects in the heart or the nervous system and are also referred to as stimulation electrodes, pacemaker electrodes, ICD electrodes, or as EP catheters (electrophysiology catheters). These comprise an elongated electrode body, which is provided with at least one electrode at or upstream of the distal end thereof. The aforementioned electrode can be, for example, a sensing electrode for the detection of cardiological or neuronal signals, an ablation electrode for the local sclerosing of cardiac tissue, or a therapy electrode for emitting electrical stimulating signals, e.g., of a neurostimulator, a cardiac pacemaker, or a defibrillator. The electrode(s) is/are provided, in each case, with an electrode lead for the electrical connection thereof to a related base device, such as an electrical generator, an electrotherapy device, or an implant, such as a neurostimulator, a cardiac pacemaker, or a defibrillator.

Typical electrode devices of the type known in diverse embodiments thereof from the prior art use solid, metallic leads or litz wires as electrode leads, in the case of which the individual fibers are not insulated from one another.

Document EP 1 872 825 B1 makes known an electrode device, in the case of which an electrode lead for the electrical connection of the electrode is formed from a bundle of filaments arranged loosely side by side, wherein the fiber structure has an anisotropic conductivity due to the surface layer of the fibers. The specific conductivity of the electrode lead is higher by at least one order of magnitude in its longitudinal direction than in its transverse direction, and therefore the specific conductivity of the electrode lead is higher in its longitudinal direction than in its transverse direction. This makes it possible, for example, to use such electrode leads in strong magnetic fields.

The problem addressed by the invention is that of improving an electrode device for electrotherapy and/or electrodiagnosis.

Another problem addressed by the invention is that of creating an implant having such an electrode device.

The problem is solved according to the invention by the features of the independent claims. Favorable embodiments and advantages of the invention will become apparent from the further claims, the description, and the drawings.

Proposed herein is an electrode device for cardiological or neurological electrodiagnosis and/or electrotherapy, which comprises at least one elongated electrode body. This has a proximal side and a distal side, wherein the electrode body comprises at least one electrode on the distal side for establishing electrical contact to the tissue and/or blood during use, wherein at least one of the electrodes is coated, at least in sections, with a dielectrically anisotropic material.

By coating an electrode pole with a dielectrically anisotropic material, it is possible to suppress unwanted far-field signals in a targeted manner without the need to make any further changes to the electrode design. Mechanically proven concepts can therefore be implemented while simultaneously optimizing the signal-to-noise ratio.

The electrode poles for the detection of the excitation signals in the heart, for example, typically comprise a metallically conductive material. Due to the low electrical resistance of a metal, electrical field lines basically extend perpendicularly to the surface of the metal. Vectoral information on the electrical field, which could provide information about the location of the source of the potential, are therefore lost and all that remains is the potential difference, as the parameter, between the tip electrode and the ring electrode.

In particular, cardiac signals located a great distance away from the electrode poles of the electrode device are characterized by an angle relative to the electrode axis that tends to be relatively flat. In addition, the electrode device is usually affixed, via the distal region thereof, at a location of the cardiac wall, which is located near the source of the useful signals. Relative to the distal region, the desired signals are therefore mainly proximal, while the far field tends to be distal. According to the invention, the detection of signals from the far field are advantageously suppressed without the useful signals being too adversely affected by taking the vectoral character of the signals into account. This is carried out by using dielectrically anisotropic materials, the dielectric properties of which do not have the same amplitude in all three spatial directions, but rather have preferential directions having higher dielectric constants. Depending on the orientation of the preferential direction(s), orientations of the electrical field can be amplified and/or suppressed before detection and, therefore, signals from the far field can be specifically excluded by the active implant before analysis.

Advantageously, far-field signals are suppressed without this resulting in a notable reduction of the useful signals. It is thereby possible to avoid the situation in which the signals to be detected are located below the sensitivity threshold of the implant, which is coupled to the electrode device, and are therefore detected not at all or erroneously. Information on the morphology of the excitation is not lost, and the reliability of the measurement is not adversely affected. The mechanical properties of the electrode are ensured by means of a flexible range between the tip electrode and the ring electrode, since the poles of the electrodes can be separated by a sufficient extent. It is also possible to separate signals from the far field from useful signals that occur at the same time.

According to a favorable embodiment of the invention, the dielectrically anisotropic material can cover the at least one electrode, at least in sections. Advantageously, at least 20%, preferably at least 30% of the area of the electrode is covered.

The dielectrically anisotropic material can cover only a part of the electrically conductive electrode material, and /or can cover only a part of a dielectrically isotropic material.

According to a favorable embodiment of the invention, the electrode can have a stacked arrangement of electrically conductive electrode material, dielectrically isotropic material, and the dielectrically anisotropic material. Favorably, the layer thickness can be in the range of 1 µm to 20 µm, preferably 2 µm to 5 µm. By suitably adjusting the region of the electrode that is covered by the stacked arrangement, it is possible to influence a suppression of interfering far-field signals in a spatially specific manner.

According to a favorable embodiment of the invention, the electrode can have a stacked arrangement of electrically conductive electrode material and the dielectrically anisotropic material. Favorably, the layer thickness can be in the range of 1 µm to 20 µm, preferably 2 µm to 5 µm. By suitably adjusting the region of the electrode that is covered by the stacked arrangement, it is possible to influence a suppression of interfering far-field signals in a spatially specific manner.

According to a favorable embodiment of the invention, the dielectrically anisotropic material can be applied such that, within the dielectrically anisotropic material, an axis having maximum dielectric conductivity (permittivity), i.e., a maximum dielectric constant, points in the proximal direction. In particular, an angle of the axis relative to the electrode axis can be less than 90°.

According to a favorable embodiment of the invention, the dielectrically anisotropic material can be uniaxially dielectrically anisotropic, at least in sections. The directional sensitivity of the electrode arrangement can be influenced in a targeted manner.

According to a favorable embodiment of the invention, as an alternative or in addition, the dielectrically anisotropic material can be biaxially dielectrically anisotropic, at least in sections. The directional sensitivity of the electrode arrangement can be influenced in a targeted manner.

According to a favorable embodiment of the invention, the dielectrically anisotropic material can have at least one region in which the orientation of an axis having higher dielectric conductivity, as compared to the dielectric conductivity along other axes in the material, is located in the proximal direction. The directional sensitivity of the electrode arrangement can be influenced in a targeted manner.

According to a favorable embodiment of the invention, the dielectrically anisotropic material can comprise at least one crystalline material, preferably a piezoelectric material or a ferroelectric material, particularly preferably one of the compounds BaTiO₃, TiO₂. According to another aspect of the invention, an implantable body for cardiological or neurological electrodiagnosis and/or electrotherapy having an electrode device according to the invention is proposed.

Advantageously, unwanted far-field signals can be suppressed without this resulting in a notable reduction of the desired useful signals. It is thereby possible to avoid the situation in which the signals to be detected are located below the sensitivity threshold of the implant and are detected not at all or erroneously.

The invention is particularly suited for use with active implants, such as pacemakers, defibrillators, and the like.

The invention is explained in the following in greater detail, as an example, with reference to exemplary embodiments that are depicted in drawings. In schematic depictions:
- Fig. 1: shows a schematic diagram of a bipolar electrode device according to one exemplary embodiment according to the invention;
- Fig. 2: shows a schematic diagram of a tip of an electrode body having field lines from a remote point source;
- Fig. 3: shows a schematic diagram of a material having dielectrically uniaxial anisotropy;
- Fig. 4: shows a schematic diagram of an electrode body of a bipolar electrode device comprising a partially dielectrically anisotropically coated ring electrode according to one exemplary embodiment of the invention;
- Fig. 5: shows a depiction of the effect on a signal amplitude with various embodiments of a ring electrode of a bipolar electrode device as a function of different positions of stationary point sources; and
- Fig. 6: shows a depiction of the effect on a signal amplitude with various embodiments of a ring electrode of a bipolar electrode device as a function of a tangential separation from an electrode tip of a point source that continuously moves along the electrode body.

Elements that are functionally identical or similar-acting are labelled using the same reference symbols in the figures. The figures are schematic depictions of the invention. They do not depict specific parameters of the invention. Furthermore, the figures merely show typical embodiments of the invention and are not intended to limit the invention to the embodiments shown.

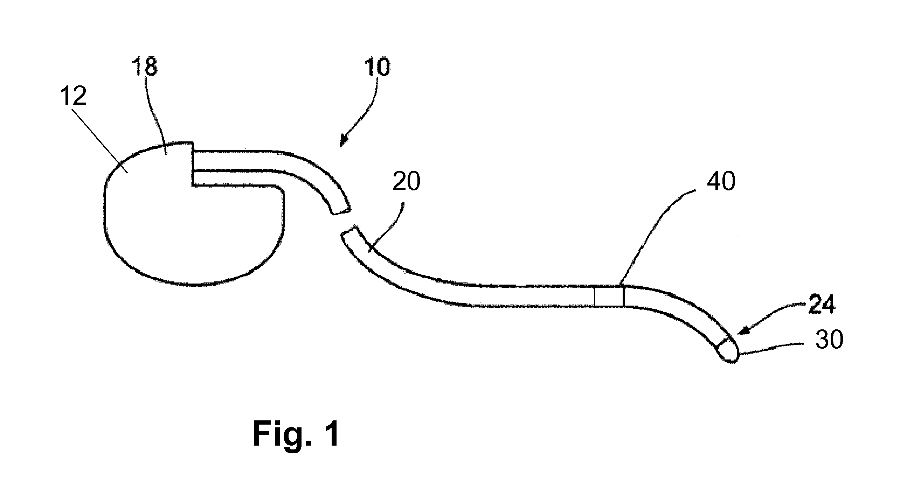

Figure 1 schematically shows an electrode device 10 in the form of a functional electrostimulation device. The electrode device 10 comprises an elongated electrode body 20 between a proximal region 18 and a distal region 24 of the electrode device 10. At the distal end 24, the electrode device 10 comprises two electrodes or electrode poles, namely a tip electrode 30 and, disposed in the proximal direction relative thereto, a ring electrode 40.

The tip electrode 30 and the ring electrode 40 are used to detect cardiac signals and to deliver stimulation pulses to surrounding tissue. The electrostimulation device also comprises a housing 12, which contains the components that are required for the functionality of the electrostimulation device, such as a pulse generator, electrical switches, and a power source.

The electrode body 20 comprises structures, which are not shown here, on the proximal end thereof, i.e., the end 18 connected to the housing 12, wherein said structures are used to establish a connection to the housing 12. Such structures are sufficiently known from the prior art and do not have any further significance in the context of the present invention, and so an extensive description thereof is omitted here.

The tip and ring electrodes 30, 40, respectively, are electrically conductive structural elements, which have a transition point for electrical energy to the cardiac tissue. The ring electrode 40 can be made of a platinum-iridium alloy, while the distal tip electrode 30 can have a hemispherical head made of an iridium-coated platinum-iridium alloy. The electrodes 30, 40 can be designed as tapping electrodes, stimulation electrodes, or measurement electrodes, and can vary to a great extent in terms of material, number, position, and geometry.

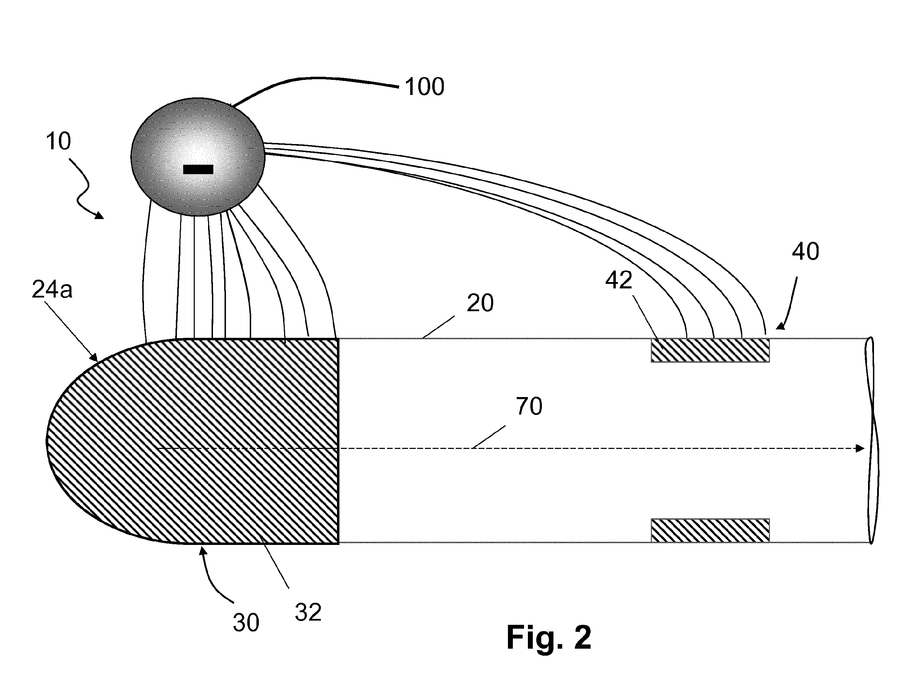

Figure 2 shows the field conditions on a known electrode device 10 for the detection of excitation signals, e.g., in the heart. Shown in the figure is a distal region 24 of a bipolar electrode device 10, on which an electrical field of a remote, idealized signal source acts, wherein said signal source is assumed to be a point source 100. The electrical field is indicated by lines, which are intended to symbolize electrical field lines and extend from the point source 100 to the electrode poles. The electrode poles are formed, for example, by a tip electrode 30 on the outermost distal end 24 of the electrode body 20 of the electrode device 10 and a ring electrode 40, which is offset in the proximal direction relative thereto and annularly encloses the electrode body 20.

The electrode poles 30, 40 are typically made of a metallically conductive material 32, 42. Due to the low electrical resistance of a metal, electrical field lines basically extend perpendicularly to the surface of the metal. Vectoral information on the electrical field, which could provide information about the location of the source of the potential (point source 100 in this case), is therefore lost and all that remains is the potential difference, as the parameter, between the electrode poles, which are the tip electrode 30 and the ring electrode 40 in this case.

In particular, cardiac signals located a great distance away from the electrode poles of the electrode device 10 are usually characterized, however, by an angle relative to the electrode axis 70 that tends to be relatively flat, as compared to useful signals from the close range of the electrodes 30, 40. In addition, the electrode device 10 is usually affixed, via the distal region 24 thereof, at a location of the cardiac wall (not shown), which is located near the source of the useful signals. Relative to the distal region 24, the desired electrical signals are therefore mainly proximal, while the far field of unwanted signals from remote sources tends to be distal.

Advantageously, the detection of signals from the far field can be suppressed or at least reduced as compared to proximal useful signals without affecting the useful signals too adversely by taking the vectoral character of the signals into account. This is carried out according to the invention by using dielectrically anisotropic materials, the dielectric properties of which do not have the same amplitude in all three spatial directions, but rather have one or more preferential directions having higher dielectric conductivity or permittivity. Depending on the orientation of the preferential direction, orientations of the electrical field can be amplified and/or suppressed before detection and, therefore, signals from the far field can be specifically excluded by the electrode device 10 before analysis.

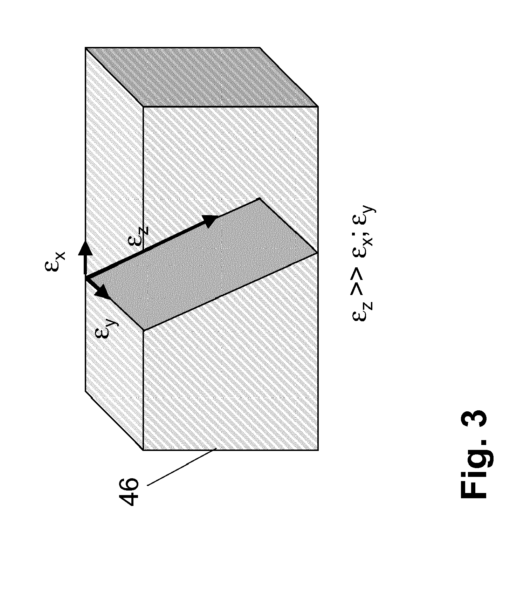

Figure 3 illustrates the relations in a material 46 having dielectrically uniaxial anisotropy of the dielectric conductivity. Three permittivity spatial components εₓ, ε_{y}, ε_{z}, instead of one spatially constant permittivity ε, are present in the three spatial directions x, y, z. In the example shown, the dielectric constants εₓ, ε_{y} are much smaller than the component ε_{z}.

In a dielectrically biaxial anisotropy, one component is smaller than the other two spatial components of the permittivity.

All crystalline materials are basically feasible, since crystals typically have an anisotropy, and materials that exhibit a piezoelectric effect and/or ferroelectricity are basically feasible, since these generally also have dielectrically anisotropic properties.

Otherwise, isotropic dielectric materials can also be converted into an anisotropic variant by applying electrical fields or mechanical stresses during the production process.

It is advantageous to use BaTiO₃, TiO₂ as the dielectrically anisotropic material 46.

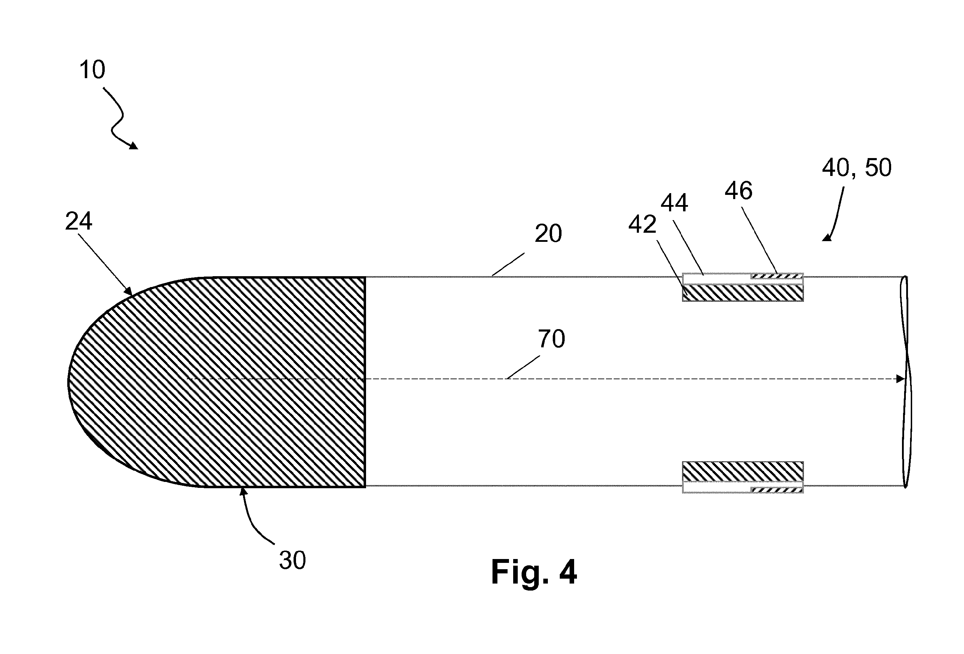

Figure 4 shows a schematic diagram of a tip of an electrode body 20 of a bipolar electrode device 10 (see Figure 1) according to one exemplary embodiment of the invention comprising a tip electrode 30 and a ring electrode 40 on an elongated electrode body 20. The ring electrode 40 is partially coated with a dielectrically anisotropic material 46.

In an electrically active medical implant, such as a stimulation device as shown in Figure 1, the electrically active pole 30 for the detection of the cardiac excitation signals is sensitized in a directionally dependent manner by means of one or more layers with dielectrically anisotropic materials 46 such that unwanted distal cardiac signals from the far field are suppressed without adversely affecting the desired detection of proximal signals (useful signals).

By coating one or both electrode poles 30, 40, at least the ring electrode 40, with dielectrically anisotropic materials 46, the electrical fields are amplified differently depending on the direction thereof such that a selective detection of the signals is made possible, which simplifies the signal processing in the active implant.

The ring electrode 40 of the electrode body 12 is coated with a layer of dielectrically isotropic material 46. The dielectrically anistropic layer is applied on the proximal side of the ring electrode 40 on an isotropic dielectric layer with material 44, wherein the preferential axis, in which the dielectric conductivity is higher, extends in the proximal direction, i.e., away from the tip electrode 30. The additional isotropic coating with material 44 underneath the anisotropic layer with the material 46 is advantageous, since the field lines extend perpendicularly to the surface of the conductor material 42 at the latest in the immediate surroundings of the metallic ring electrode 40 and the advantages of a directionally selective field suppression were reduced by means of a direct application of a dielectrically anisotropic layer. Barium titanate (BaTiO3) is preferable as the dielectrically anisotropic material 46 having one of the highest anisotropy values while also being absolutely biocompatible.

Dielectrically isotropic material 44 can also be disposed between the dielectrically anisotropic material 46 and the metallic material 42 of the electrode 40 such that a stacked arrangement 50 of metallic material 42, dielectrically isotropic material 44, and dielectrically anisotropic material 46 is formed.

In this connection, the dielectrically anisotropic material 46 in one exemplary embodiment can cover only a portion of the metallic conductor material 42.

As an alternative, the dielectrically anisotropic material 46 can cover only a portion of the dielectrically isotropic material 44 disposed over the metallic conductor material 42.

The dielectrically anisotropic material 46 can be designed to be uniaxially dielectrically anisotropic or biaxially dielectrically anisotropic.

Advantageously, within the dielectrically anisotropic material 46, the orientation of the axis having the higher dielectric conductivity is located in the proximal direction such that the angle of the axis having higher permittivity as compared to the other two spatial components of the permittivity relative to the electrode axis 70 is less than 90°.

By coating an electrode pole 30, 40 with a dielectrically anisotropic material 46, it is possible to suppress unwanted far-field signals in a targeted manner without the need to make any further changes to the electrode design. Mechanically proven concepts can therefore be implemented while simultaneously optimizing the signal-to-noise ratio.

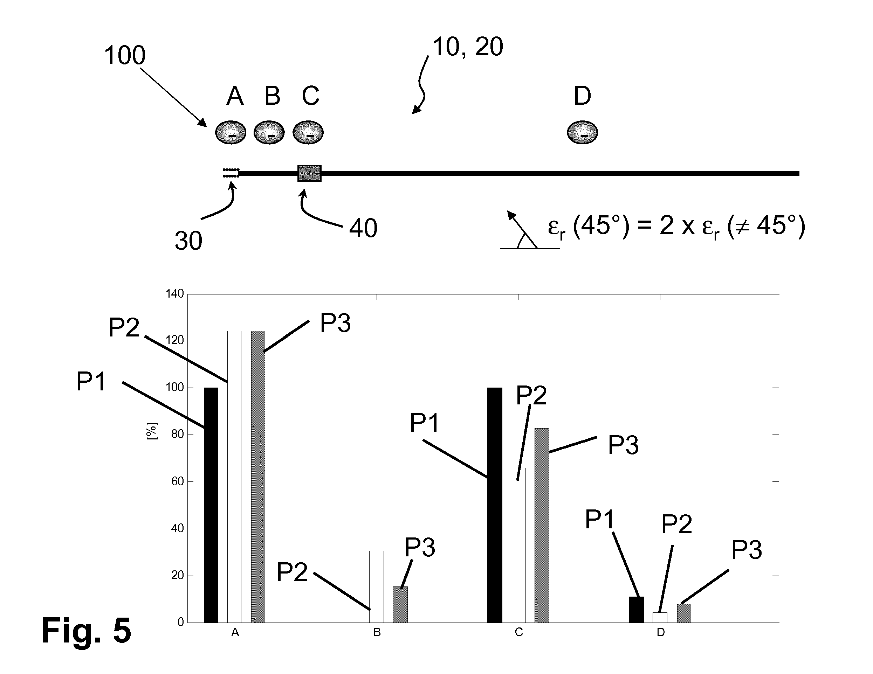

Figure 5 shows a depiction of the effect on a signal amplitude with various designs of a ring electrode 40 of a bipolar electrode device 10 (see, e.g., Figure 1) as a function of different positions A, B, C, D of stationary point sources 100 along the electrode body 20. The point sources 100 are signal sources having an unwanted signal that is intended to be attenuated.

The plot shows the standardized potential difference in percent as a function of the position A, B, C, D of the point sources 100 along the electrode body 20 (Figure 1) for three different embodiments P1, P2, P3 on the ring electrode 40.

Position A represents a position at the level of the tip electrode 30. Position B is a position in the center between the tip electrode and the ring electrode 30, 40, respectively. Position C is a position at the level of the ring electrode 40 (1 cm away from the tip electrode 30). Position D is located 5 cm away from the tip electrode 30 and 4 cm away from the ring electrode 40. The point source 100 is located 1 cm away from the electrode body 20 in each case. The medium in which the electrode poles 30, 40 are located is a dielectric material having a relative permittivity of εᵣ =10, corresponding to tissue or blood, for example.

P1 represents the ring electrode 40 without a dielectrically anisotropic coating. P2 represents a layer with an isotropic dielectric material having a permittivity ε, which reduces the electrical potential of the field belonging to the source, at the electrode 40 by 10%. P3 represents a sequence of anisotropic dielectric material having a permittivity ε, which reduces the electrical potential by 10% in the direction 45° in the distal direction. For an angle that is not 45°, the potential is reduced by only 5%. The potential differences were standardized, in each case, to the potential difference at position A, having an uncoated ring electrode 40 (P1).

The aforementioned values are merely examples and can vary in other geometric and material embodiments of the electrode unit 10.

If only the amplitudes of the far field are considered, the effect of the isotropic coating is initially greater. However, desired useful signals are also suppressed by a greater extent, said useful signals being located in the immediate surroundings of the electrode pole, but not unambiguously proximally. Useful signals that originate laterally relative to the electrode head, but which are near-field, are suppressed by a lesser extent with an anisotropic coating.

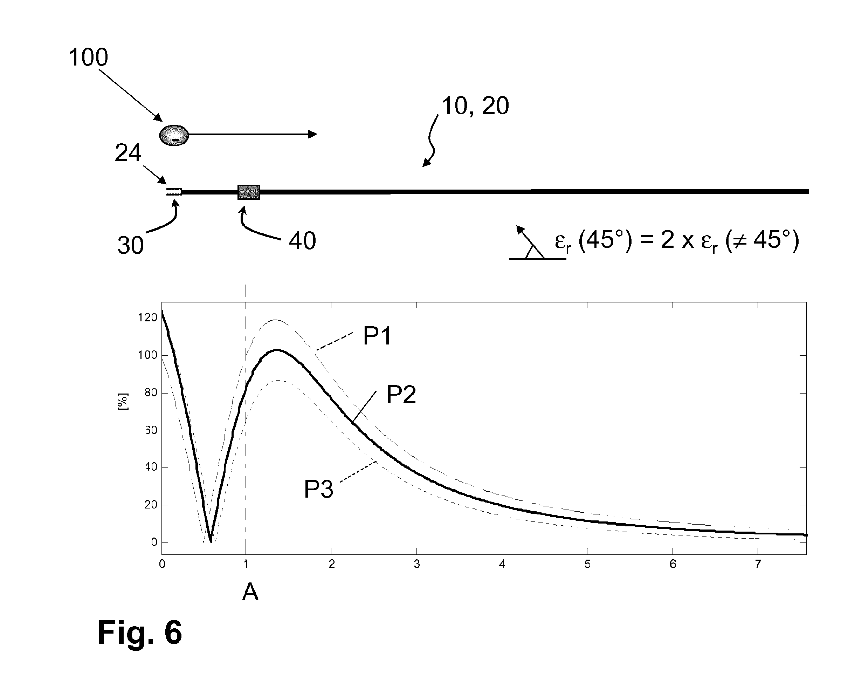

Figure 6 shows a depiction of the effect on a signal amplitude with various embodiments of a ring electrode 40 of a bipolar electrode device 10 as a function of a tangential separation from an electrode tip (tip electrode 30) of a point source 100 that continuously moves along the electrode body 20.

Instead of the discrete positions A, B, C, D, the position of the point source 100 was continuously moved along the electrode body 10 and the position of the ring electrode 40 was held fixed. The point source 100 is located at the level of the tip electrode 30 (corresponding to position A in Figure 5) with a spacing of 1 cm. The medium in which the point source 100 and the electrode body 20 are located is a dielectric material having a relative permittivity of εᵣ =10, corresponding to tissue or blood, for example.

P1 represents the ring electrode 40 without a dielectrically anisotropic coating. P2 represents a layer with an isotropic dielectric material having a permittivity ε, which reduces the electrical potential of the field belonging to the source, at the electrode 40 by 10%. P3 represents a sequence of anisotropic dielectric material having a permittivity ε, which reduces the electrical potential by 10% in the direction 45° in the distal direction. For an angle that is not 45°, the potential is reduced by only 5%. The potential differences were standardized, in each case, to the potential difference at position A, having an uncoated ring electrode 40 (P1).

Figures 5 and 6 respectively show the excessive increase in the potential difference with a coated ring electrode 40 (P2, P3) in the distal region 24 of the tip electrode pole 30 as compared to an uncoated ring electrode 40 (P1). The far field for positions of the point source 100 located more than 1 cm away from the tip electrode 30 in the proximal direction is suppressed by the coating P3, P2 as compared to an uncoated ring electrode 40 (P1).

## Claims

1. An electrode device (10) for cardiological or neurological electrodiagnosis and/or electrotherapy, comprising at least one elongated electrode body (20), which has a proximal side (18) and a distal side (24), wherein the electrode body (20) comprises at least one electrode (30, 40) on the distal side (24) for establishing electrical contact to the tissue and/or blood during use, wherein at least one of the electrodes (30, 40) is coated, at least in sections, with a dielectrically anisotropic material (46).

2. The electrode device according to claim 1, wherein the dielectrically anisotropic material (34) covers the at least one electrode (40), at least in sections.

3. The electrode device according to claim 1 or 2, wherein the electrode (40) has a stacked arrangement (50) of electrically conductive electrode material (42), dielectrically isotropic material (46), and the dielectrically anisotropic material (46).

4. The electrode device according to any one of the preceding claims, wherein the electrode (40) has a stacked arrangement (50) of electrically conductive electrode material (42) and the dielectrically anisotropic material (46).

5. The electrode device according to any one of the preceding claims, wherein the dielectrically anisotropic material (46) is applied such that, within the dielectrically anisotropic material (46), an angle of an axis having higher dielectric conductivity points in the proximal direction (70).

6. The electrode device according to claim 5, wherein the angle relative to the electrode axis is less than 90°.

7. The electrode device according to any one of the preceding claims, wherein the dielectrically anisotropic material (46) is uniaxially dielectrically anisotropic, at least in sections.

8. The electrode device according to any one of the preceding claims, wherein the dielectrically anisotropic material (46) is biaxially dielectrically anisotropic, at least in sections.

9. The electrode device according to any one of the preceding claims, wherein the dielectrically anisotropic material (46) has at least one region in which the orientation of an axis having higher dielectric conductivity, as compared to the dielectric conductivity along other axes in the material, is located in the proximal direction.

10. The electrode device according to any one of the preceding claims, wherein the dielectrically anisotropic material (46) comprises at least one crystalline material, preferably a piezoelectric material or a ferroelectric material, particularly preferably one of the compounds BaTiO₃, TiO₂.

11. An implantable body for cardiological or neurological electrodiagnosis and/or electrotherapy, comprising an electrode device (10) according to any one of the preceding claims.
